# FASCICULE DE BREVET EUROPEEN

(11) **EP 0 740 943 B1**
(45) Date de publication et mention de la délivrance du brevet: **02.01.2003**
(21) Numéro de dépôt: 96450007.8
(22) Date de dépôt: 26.04.1996
(51) Int. Cl.: A61N 1/05

(54) **Dispositif de maniement et de mise en place d'une électrode d'appareil de stimulation cardiaque**
Vorrichtung zur Handhabung und zum Einsetzen einer Elektrode eines Herzschrittmachergeräts
Handling and positioning device for an electrode of a pacemaker apparatus

(30) Priorité: 28.04.1995 FR 9505363
(43) Date de publication de la demande: 06.11.1996
(73) Titulaire: SOCIETE ETUDES ET DEVELOPPEMENTS S.E.D., F-19130 Objat (FR); ELA MEDICAL (Société anonyme), F-92541 Montrouge (FR)
(72) Inventeur: Jammet, Jean, 19130 Voutezac (FR); Peyre, André, 33000 Bordeaux (FR)
(74) Mandataire: Thébault, Jean-Louis

(56) Documents cités:
- EP-A- 0 282 047
- EP-A- 0 491 082
- EP-A- 0 493 221

## Description

La présente invention a pour objet un dispositif de maniement et de mise en place d'une électrode d'appareil de stimulation cardiaque.

Un dispositif de maniement et de mise en place d'une électrode d'appareil de stimulation cardiaque à l'aide d'un stylet est connu du document EP-A-0 491 082.

On connaît par la demande de brevet EP-A-591 053 au nom du présent demandeur, une électrode à vis biologique rétractable pour appareil de stimulation cardiaque. Cette électrode donne la plus grande satisfaction pour le patient et sur le plan de la conception. On se reportera de façon utile au texte pour une description détaillée et un fonctionnement complet mais d'ores et déjà il convient de donner quelques indications nécessaires à la mise en valeur du présent dispositif et à la compréhension de son fonctionnement.

L'électrode comprend un corps creux, une vis mécanique interne avec un pas à gauche, coopérant avec un filetage conjugué ménagé à l'intérieur du corps et une vis biologique, solidaire de cette vis mécanique, dont le pas est à droite.

La vis biologique est effilée à son extrémité libre de façon à pénétrer aisément dans les tissus. La vis biologique peut prendre deux positions, l'une rétractée dans laquelle elle est entièrement logée dans le corps de l'électrode et l'autre, en saillie, dans laquelle elle est en grande partie en saillie à l'extérieur du corps de ladite électrode.

Dans le cas général, il faut manoeuvrer avec doigté afin de ne pas forcer le vissage de la vis biologique et de ne pas arracher le tissu. Dans le cas de l'électrode de la demande européenne précitée, il est prévu une gorge "point mort" qui évite toute rotation supplémentaire de la vis biologique, rédhibitoire pour l'intervention car elle génère des dégâts physiologiques.

Le corps de l'électrode est fixé à un ressort à spires jointives de très faible diamètre, en métal conducteur, entouré d'une gaine isolante en matériau polymère biocompatible.

L'extrémité libre du ressort comprend un connecteur prévu pour être branché sur le stimulateur cardiaque.

De façon connue, le praticien introduit l'électrode par la veine jusque dans la cavité concernée du coeur et il doit déterminer le site le plus approprié tant sur le plan électrique que sur le plan de la qualité des tissus.

A cet effet, il faut généralement effectuer plusieurs essais.

De façon générale, le praticien doit tout d'abord mettre en saillie la vis biologique par rapport au corps de l'électrode. Pour y parvenir, il introduit un stylet de manoeuvre de la vis mécanique dans le ressort gainé et, tout en maintenant le stylet immobile, il fait tourner le ressort dans le sens de vissage d'un pas à droite, plus conforme aux gestes naturels, car le pas de la vis mécanique dans le corps de l'électrode est un pas à gauche.

On constate que la gorge "point mort" joue son rôle puisque le praticien ne peut pas faire d'erreur d'appréciation de la mise en saillie de la vis biologique.

En cas de doute, il peut faire subir un tour supplémentaire au ressort, sans aucun risque, puisque la vis mécanique échappe dans la gorge "point mort".

La vis mécanique et le corps de l'électrode sont liés l'un à l'autre par des forces de frottement, notamment celles dues à l'ergot dans le filetage, au ressort et à la vis biologique dans les différents joints. Ces forces de frottement sont calculées pour soutenir un couple donné, suffisamment faible pour ne pas engendrer de dégradations du tissu dans lequel la vis biologique est vissée.

Le praticien retire le stylet de manoeuvre de la vis mécanique et introduit un stylet de conformation qui ne vient pas en prise avec la vis mécanique mais dont le rôle est de guider et, éventuellement, de courber le ressort afin d'implanter l'électrode sur un site recherché tout en permettant au praticien de plaquer axialement l'électrode contre la paroi interne de la cavité cardiaque.

Il convient alors, tout en maintenant fixe en rotation le stylet de conformation, de faire tourner la vis biologique pour qu'elle amorce le vissage et le poursuive jusqu'à implantation complète. Le stylet de conformation doit rester fixe en rotation car toute rotation provoquerait un déplacement angulaire de l'électrode qui conduirait à un piquage de la vis biologique en un site autre que celui retenu.

A cet effet, le praticien doit faire tourner le ressort gainé, ce qui, par l'effet des forces de frottement entre le corps de l'électrode et la vis mécanique à laquelle est fixée la vis biologique, entraîne en rotation ladite vis biologique, tout en maintenant le stylet fixe en rotation pour les raisons évoquées ci-avant.

On remarque l'intérêt de la gorge "point mort" quand elle est présente comme dans le mode de réalisation décrit dans la demande de brevet précitée. Elle permet au praticien de tourner le corps de l'électrode au-delà de ce qui est nécessaire sans pour autant provoquer le déchirement du tissu, car les frottements entre le corps de l'électrode et la vis mécanique deviennent insuffisants pour entraîner cette vis mécanique. En effet, de l'autre côté, la vis mécanique est immobilisée par la vis biologique entièrement implantée. La vis mécanique se trouve débrayée par rapport au corps de l'électrode dans la gorge "point mort".

Dans la pratique, on s'est aperçu par le retour d'expérience des utilisateurs que, si l'électrode donne entière satisfaction pour le patient et pour le praticien du point de vue du résultat, le maniement successif des deux stylets en rotation ou en blocage, tout en faisant tourner le ressort solidaire du corps de l'électrode, est relativement délicat, surtout si l'on ajoute que le ressort se comprime avec des tours morts lors de la rotation et qu'il faut exercer simultanément une pression axiale.

Aussi, le procédé de mise en place et l'électrode à vis rétractable présentent les meilleures garanties pour le patient et pour le praticien mais la mise en oeuvre ne doit pas remettre en cause ces garanties et les avantages qui en découlent.

Le but de l'invention est de proposer un dispositif de maniement qui facilite grandement la mise en oeuvre d'une électrode à vis rétractable, notamment à gorge "point mort", qui soit d'une grande simplicité et d'un prix de revient suffisamment peu élevé pour qu'il puisse être fabriqué en matière plastique de façon qu'il soit à usage unique si besoin est, qui soit d'une totale fiabilité en ne faisant appel à aucune mécanique tournante complexe et qui puisse être aisément stérilisé par les méthodes actuellement utilisées.

A cet effet, selon l'invention, le dispositif de maniement et de mise en place d'une électrode d'appareil de stimulation cardiaque à l'aide d'au moins un stylet, ladite électrode comprenant un corps, une vis mécanique se vissant dans ledit corps, une vis biologique solidaire de la vis mécanique pouvant prendre une première position à l'intérieur du corps et une seconde position en saillie, un ressort gainé de liaison fixé à l'électrode et comportant un connecteur à son extrémité libre, se caractérise en ce qu'il comprend un corps préhensible équipé de moyens d'accouplement pour recevoir et pour bloquer le connecteur du ressort gainé, des moyens pour faire tourner lesdits moyens d'accouplement par rapport audit corps préhensible et des moyens de réception pour permettre l'introduction du stylet utilisé dans le ressort gainé et pour bloquer en translation et en rotation ledit stylet par rapport audit corps préhensible en sorte de permettre un blocage temporaire en translation et en rotation dudit stylet par rapport audit ressort.

Selon une autre caractéristique, les moyens d'accouplement comprennent un manchon d'accouplement fixé sur un axe creux, intérieur et coaxial au corps préhensible, cet axe creux étant solidaire d'une molette de manoeuvre accessible de l'extérieur du corps préhensible.

Il est en outre prévu des moyens de rappel élastique destinés à tirer l'axe creux de l'aval vers l'amont en sorte de plaquer le manchon d'accouplement contre le corps en exerçant un freinage en rotation de ce manchon.

Plus particulièrement, le ressort est monté coaxial à l'axe creux et prend appui d'un côté sur la molette de manoeuvre et de l'autre sur une bague dont la surface de contact avec l'intérieur du corps préhensible est réduite.

Selon une caractéristique importante de l'invention, le dispositif comprend des moyens détrompeurs du sens de rotation et de comptage.

Ces moyens détrompeurs comprennent un ergot en saillie à l'intérieur du corps et venu de fabrication avec ce corps, et une lame élastique solidaire de la molette de manoeuvre prévue pour venir cliqueter sur ledit ergot.

Selon un mode de réalisation préférentiel de l'invention, les moyens de réception comprennent un U dont les branches reçoivent un bouton de préhension d'un stylet, et un entonnoir, disposé entre les branches, pour faciliter l'introduction du fil de ce même stylet.

La présente invention est décrite ci-après en regard des dessins annexés sur lesquels les figures suivantes représentent un mode de réalisation particulier non limitatif, plus particulièrement :
- la figure 1, montre une vue en perspective schématique du dispositif de maniement avec une sonde cardiaque complète à l'exception du stimulateur, selon un premier mode de réalisation,
- la figure 2, montre une vue en coupe par un axe longitudinal médian du dispositif selon l'invention,
- la figure 3, montre une vue en coupe transversale selon la ligne 3-3 de la figure 2,
- la figure 4, montre une vue en coupe transversale selon la ligne 4-4 de la figure 2,
- la figure 5, montre une vue en coupe transversale selon la ligne 5-5 de la figure 2,
- la figure 6, montre une vue en coupe transversale selon la ligne 6-6 de la figure 2,
- la figure 7, montre une vue en coupe transversale selon la ligne 7-7 de la figure 2,
- la figure 8, montre une vue en coupe transversale selon la ligne 8-8 de la figure 2,
- la figure 9, montre une vue en coupe transversale selon la ligne 9-9 de la figure 2,
- la figure 10, montre une vue en coupe transversale selon la ligne 10-10 de la figure 2,
- la figure 11, montre une vue en coupe transversale selon la ligne 11-11 de la figure 2,
- les figures 12A à 12E montrent un synoptique de mise en oeuvre du dispositif selon l'invention au cours de l'implantation d'une électrode,
- la figure 13 montre une vue d'un mode de réalisation préférentiel de l'invention, et
- les figures 14A à 14E montrent les différentes étapes de mise en oeuvre de cette variante.

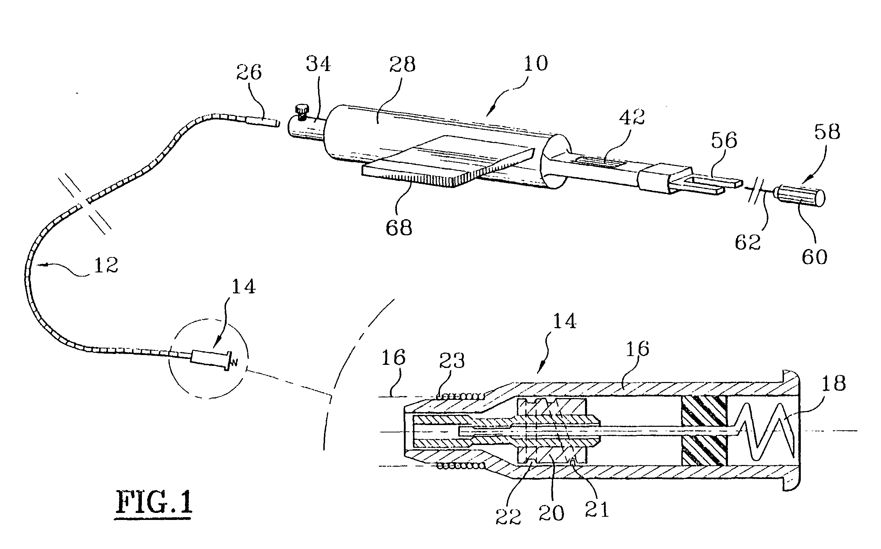

Sur la figure 1, on a représenté une vue en perspective du dispositif de maniement 10 selon l'invention, un ressort gainé de liaison 12 et une électrode 14 à vis rétractable, notamment du type de celle décrite et revendiquée dans la demande de brevet européen EP-A-591 053.

Cette électrode comprend un corps 16, une vis biologique avec un pas à droite 18 solidaire d'une vis mécanique avec un pas à gauche 20, cette vis mécanique coopérant avec un ergot 21 formant un filetage à pas conjugué, ménagé dans le corps de ladite électrode. La vis mécanique comprend une gorge "point mort" 22.

Le corps est solidarisé au ressort gainé de liaison qui comprend un ressort 23 métallique à spires jointives, réalisé à partir d'un fil de petit diamètre ce qui lui donne une certaine souplesse et dont le corollaire est une certaine propension à générer des tours morts lorsqu'il est mis en rotation à une extrémité avant de transmettre le couple à son autre extrémité et les fournisseurs de tels ressorts sont à même de fournir les ressorts adaptés pour un meilleur compromis.

La gaine 24 participe à la raideur de l'ensemble et doit être étudiée dans ce sens mais elle permet surtout d'isoler électriquement le ressort par lequel passent les impulsions électriques générées par le stimulateur, afin que le courant se propage bien vers l'extrémité du corps de l'électrode au contact du tissu.

L'extrémité libre du ressort comprend un connecteur 26, prévu pour être branché sur le stimulateur (non représenté).

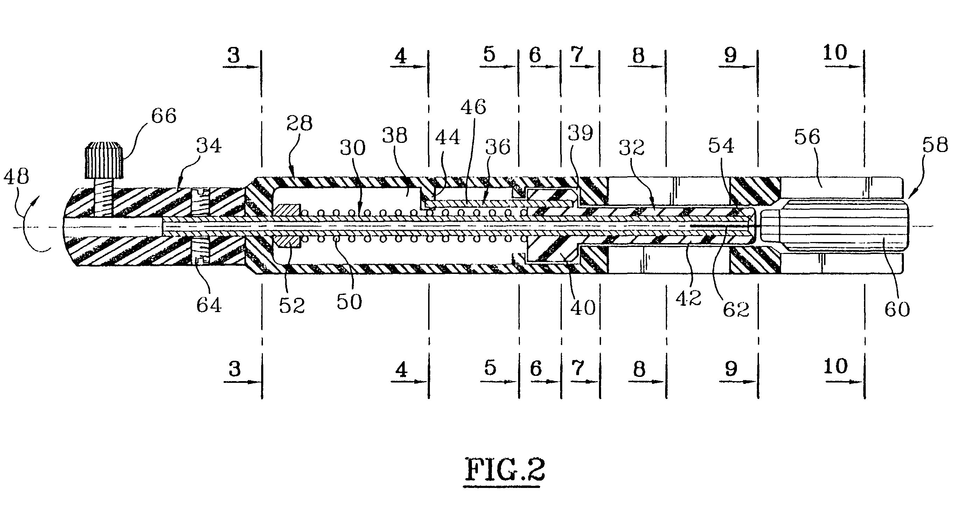

Ainsi que représenté également sur la coupe de la figure 2, le dispositif de maniement comprend un corps tubulaire 28, un axe creux 30 monté coaxial, tourillonnant par rapport à l'axe dudit corps, des moyens de manoeuvre 32 de l'axe creux, un manchon d'accouplement 34 et des moyens détrompeurs 36.

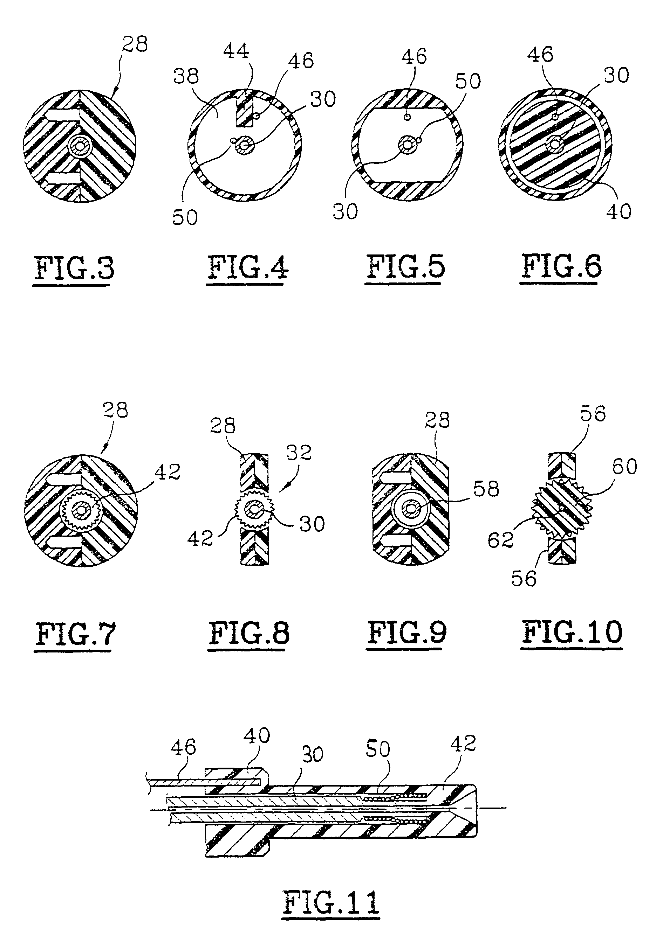

Le corps comprend trois parties I, Il, III, et est réalisé à partir de deux demi-coquilles. La partie I, comprise sensiblement entre les traits de coupe 3-3 et 7-7, est une chambre 38, traversée par l'axe creux 30 qui comprend un premier logement 39 prévu pour recevoir une partie des moyens de manoeuvre 32 de l'axe creux sous forme d'une embase 40, figure 6, d'une molette de manoeuvre 42, figure 7, se prolongeant jusqu'à la partie II.

Cette chambre 38 comprend en outre un ergot 44, en saillie vers l'intérieur, et une lame de ressort 46, solidaire de l'embase 40 des moyens de manoeuvre 32, ladite lame venant en contact avec l'ergot à chaque tour comme cela sera expliqué ultérieurement.

La forme de l'ergot est avantageusement à pente douce dans le sens de rotation, pas à droite, tel qu'indiqué par la flèche 48 sur la figure 2 et à pente raide dans le sens contraire.

Un ressort de rappel 50 est monté coaxial sur l'axe creux 30, l'une des extrémités venant en butée sur l'embase 40 et l'autre extrémité venant en butée sur une bague 52 coulissante sur l'axe creux et en appui sur l'extrémité intérieure de la chambre 38. Cette bague a une surface de contact réduite. On se reportera utilement aux coupes des figures 3 à 7 pour les détails, les références portées étant identiques à celles des figures 1 et 2.

La deuxième partie II, sensiblement comprise entre les traits de coupe 7-7 et 9-9 comprend les moyens de manoeuvre 32 avec la molette de manoeuvre 42 qui est accessible de l'extérieur par la forme rétrécie du corps. Cette molette de manoeuvre 42 est solidaire de l'axe creux 30. Elle comprend un entonnoir de guidage 54 à son extrémité. On se reportera utilement aux coupes des figures 8 et 9 pour les détails, les références portées étant identiques à celles des figures 1 et 2.

La troisième partie III, qui est l'extrémité du dispositif, comprend deux ailes 56, formant un U. Sur les différentes figures, on a représenté un stylet 58 avec un bouton de préhension 60 et un fil 62. Pour des raisons pratiques, on a représenté une toute petite longueur de fil, mais celui-ci se prolonge de façon connue et nécessaire jusqu'à la vis mécanique.

Le manchon d'accouplement 34 est creux et reçoit l'extrémité débouchante de l'axe creux 30 traversant. Deux vis de blocage 64, sans tête, permettent de solidariser l'axe creux et le manchon. Une vis 66 à tête moletée pour faciliter la manoeuvre manuelle, permet également de bloquer un autre corps cylindrique engagé dans ledit manchon.

Le corps du dispositif est en outre complété par une poignée 68.

Sur la figure 11, on trouve une variante d'agencement de l'embase 40 et du ressort 50 par rapport à l'arbre creux 30.

La mise en oeuvre du dispositif par un praticien est détaillée ci-après.

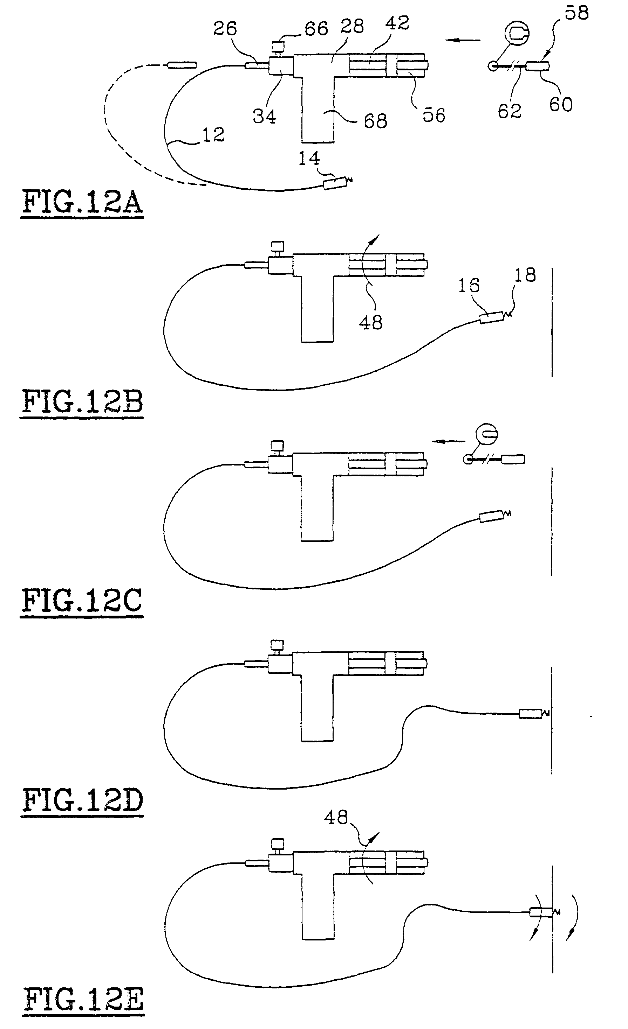

Le connecteur 26 est introduit dans le manchon d'accouplement 34 puis bloqué par la vis moletée 66, le corps étant tenu à la main par le praticien grâce à la poignée 68. De ce fait, le praticien doit se placer du bon côté suivant qu'il est droitier ou gaucher, le dispositif étant utilisable de la même façon dans les deux cas (voir figure 12A).

La partie fil 62 du premier stylet 58 est introduite dans l'axe creux 30 jusqu'à l'électrode. On remarque que le premier stylet utilisé comprend un embout type palette, voir la loupe, afin de coopérer avec la vis mécanique et assurer son blocage en rotation.

Une fois le stylet en place et enfoncé à fond, c'est-à-dire jusqu'à mettre en pression axialement le ressort gainé 12, en sorte que l'extrémité du stylet soit bien plaquée sur la vis mécanique, le bouton 60 est bloqué dans les ailes 56.

L'électrode est introduite dans la veine appropriée jusqu'à la cavité concernée du coeur, de façon connue, grâce au stylet, sans faire tourner aucune des parties l'une par rapport à l'autre afin de conserver la vis biologique dans le corps de l'électrode, évitant ainsi tout accrochage intempestif.

Une fois l'électrode arrivée au lieu choisi, le praticien tourne le bouton de manoeuvre 42 dans le sens du vissage, ce qui provoque la rotation du ressort gainé et du corps de l'électrode, alors que la vis mécanique est immobilisée par le stylet, mettant ainsi en saillie la vis biologique 18 (figure 12B). La lame de ressort lui permet à ce moment de compter le nombre de tours et, si besoin est, l'effet détrompeur, dû aux pentes de l'ergot 44, lui permet d'éviter toute erreur de sens de rotation car le sens de vissage du pas à droite est facilité tandis que le sens inverse est beaucoup plus dur.

En outre, lors de la rotation, il faut réaliser un certain nombre de tours morts pour compenser la contraction du ressort gainé et, dans ce cas, le manchon qui est plaqué contre le corps préhensible par l'effet de rappel élastique de l'aval vers l'amont, joue le rôle d'anti-retour, par freinage dû aux frottements.

On remarque à ce sujet que la bague 52 a une surface de contact la plus réduite possible avec l'intérieur de la chambre 38, de façon que l'effort de rappel du ressort s'exerce principalement sur le manchon.

Le praticien change ensuite de stylet (voir figure 12C) et introduit un stylet permettant de pousser le corps de l'électrode mais sans immobiliser en rotation la vis mécanique.

Ce stylet lui permet de conduire l'électrode sur le site retenu comme indiqué sur la figure 12D, en sorte que la vis biologique 18 en saillie vienne au contact du tissu dans lequel elle doit être implantée.

Le praticien tourne à nouveau la molette de manoeuvre 42 dans le sens de vissage d'un pas à droite comme l'indique la flèche. Cela a pour conséquence de faire tourner le corps de l'électrode, la vis mécanique et la vis biologique car les frottements entre le corps de l'électrode et la vis mécanique sont suffisants pour assurer un couple de vissage.

Dès que la vis biologique est totalement implantée par vissage et que le corps de l'électrode vient au contact du tissu, le couple résistant est supérieur aux forces de frottement et il y a débrayage de la vis mécanique par rapport au corps. On évite ainsi tout vissage violent préjudiciable à la qualité de la liaison et pouvant provoquer des dégâts physiologiques. Par contre, le praticien peut prolonger quelque peu la rotation de la molette de manoeuvre, sans aucun risque, et avec l'avantage d'avoir la certitude d'une bonne implantation.

Si le site choisi ne donne pas satisfaction, par exemple au niveau contact électrique, le praticien peut retirer la sonde par manoeuvre inverse à l'aide des différents stylets et du dispositif selon la présente invention.

Suivant les différentes applications, on remarque que le praticien n'est pas obligé de changer de stylet, notamment lorsqu'il n'y a pas de conformation nécessaire et que l'électrode est implantée dans le sens de l'introduction sans nécessiter de conformation en J pour une implantation spécifique. Il lui suffit de retirer le premier stylet sur une distance telle qu'il n'immobilise plus la vis mécanique tout en assurant le guidage.

Le dispositif selon l'invention est bien entendu utilisable avec une vis non rétractable car il faut aussi tenir un stylet immobile tout en maniant en rotation la sonde, ce qui peut être fait aisément, d'une main, par le praticien.

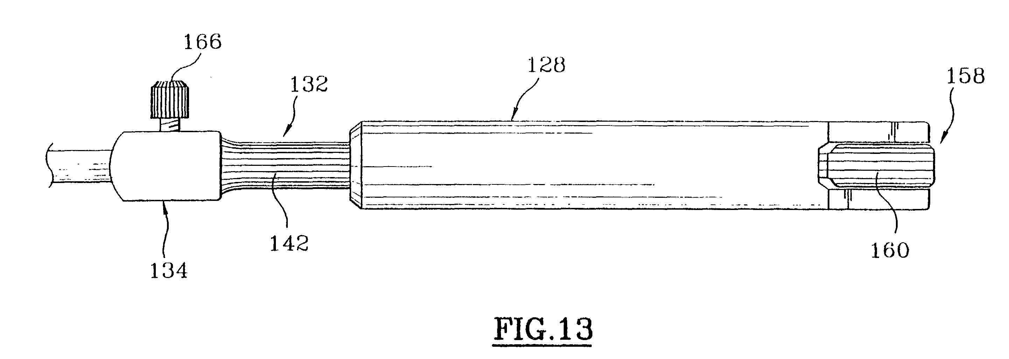

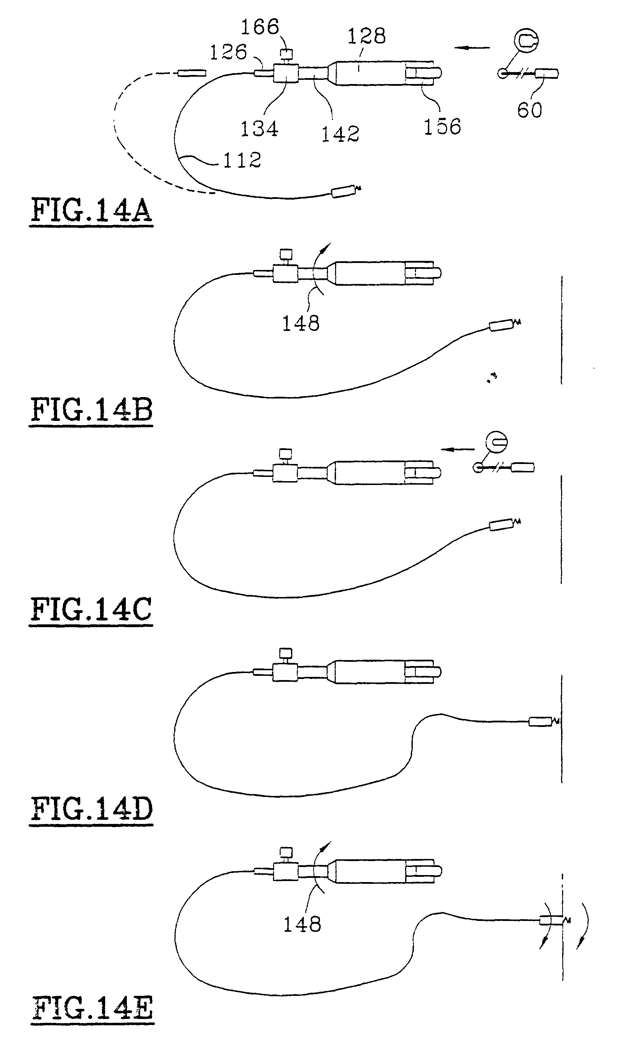

L'invention propose également un mode de réalisation préféré décrit sur les figures 13 et 14A à 14E.

Ce mode de réalisation reprend les mêmes fonctions, ce qui peut être constaté aisément à la lecture du synoptique.

Néanmoins dans cette variante, il est prévu de déplacer les moyens de manoeuvre 132, à l'extrémité du corps et plus accessibles. Tous les éléments correspondant à des éléments identiques portent les mêmes références augmentées de 100. La molette de manoeuvre 142 est venue de moulage avec le manchon d'accouplement 134, ce qui simplifie la fabrication.

Cette ergonomie facilite aussi le maniement par le praticien.

De façon préférentielle, la vis de serrage 166 sera réalisée en métal conducteur de façon que le praticien puisse connecter ladite vis à un appareillage en vue de mesurer le seuil de réponse de la sonde.

De même, un autre perfectionnement prévoit le freinage entre l'embase homologue de l'embase 40, qui n'est plus qu'une butée en appui sur la paroi intérieure du corps 128. Le freinage est ainsi protégé des agents extérieurs, comme de l'eau ou du sang, qui pourraient perturber le freinage préétabli.

## Revendications

1. Dispositif de maniement et de mise en place d'une électrode d'appareil de stimulation cardiaque à l'aide d'au moins un stylet (58), ladite électrode comprenant un corps (16), une vis mécanique (20) se vissant dans ledit corps, une vis biologique (18) solidaire de la vis mécanique pouvant prendre une première position à l'intérieur du corps et une seconde position en saillie, un ressort gainé de liaison (12) fixé à l'électrode et comportant un connecteur (26) à son extrémité libre, **caractérisé en ce qu'**il comprend un corps préhensible (28) équipé de moyens d'accouplement (34) pour recevoir et pour bloquer le connecteur (26) du ressort gainé, des moyens (42) pour faire tourner lesdits moyens d'accouplement par rapport audit corps préhensible (28) et des moyens de réception (56) pour permettre l'introduction du stylet (58) utilisé dans le ressort gainé et pour bloquer en translation et en rotation ledit stylet (58) par rapport audit corps préhensible (28) en sorte de permettre un blocage temporaire en translation et en rotation dudit stylet (58) par rapport audit ressort (12).

2. Dispositif de maniement et de mise en place d'une électrode selon la revendication 1, **caractérisé en ce que** les moyens d'accouplement comprennent un manchon d'accouplement (34) fixé sur un axe creux (30), intérieur et coaxial au corps préhensible, cet axe creux étant solidaire d'une molette de manoeuvre (42) accessible de l'extérieur du corps préhensible.

3. Dispositif de maniement et de mise en place d'une électrode selon la revendication 2, **caractérisé en ce qu'**il comprend des moyens de rappel élastique (50) prévus pour tirer l'axe creux (30) de l'aval vers l'amont en sorte de plaquer le manchon d'accouplement (34) contre le corps préhensible (28) en exerçant un freinage en rotation de ce manchon.

4. Dispositif de maniement et de mise en place d'une électrode selon la revendication 3, **caractérisé en ce que** les moyens de rappel élastique (50) sont montés coaxial à l'axe creux (30) et prennent appui d'un côté sur la molette de manoeuvre (42) et de l'autre sur une bague (52) dont la surface de contact avec l'intérieur du corps préhensible (28) est réduite.

5. Dispositif de maniement et de mise en place d'une éléctrode selon la revendication 2, **caractérisé en ce que** la molette de manoeuvre (142) vient de fabrication avec le manchon d'accouplement (134).

6. Dispositif de maniement et de mise en place d'une éléctrode selon l'une quelconque des revendications 1 à 5, **caractérisé en ce qu'**il comprend des moyens détrompeurs (36) du sens de rotation et de comptage.

7. Dispositif de maniement et de mise en place d'une éléctrode selon la revendication 6 quand dépendante des revendications 2 à 5, **caractérisé en ce que** les moyens détrompeurs comprennent un ergot (44) en saillie à l'intérieur du corps préhensible (28) et venu de fabrication avec ce corps, et une lame élastique (46) solidaire de la molette de manoeuvre (42) prévue pour venir cliqueter sur ledit ergot.

8. Dispositif de maniement et de mise en place d'une éléctrode selon l'une quelconque des revendications 2 à 7, **caractérisé en ce que** les moyens de réception comprennent un U dont les branches (56) reçoivent un bouton de préhension (60) d'un stylet (58), et un entonnoir (54), solidaire de l'axe creux, disposé entre les branches, pour faciliter l'introduction du fil (62) de ce même stylet.

9. Dispositif de maniement et de mise en place d'une électrode selon l'une quelconque des revendications précédentes, **caractérisé en ce que** les moyens d'accouplement comprennent une vis (166) métallique afin de permettre la détermination du seuil de réponse de la sonde

## Patentansprüche

1. Vorrichtung zum Handhaben und Anordnen einer Elektrode eines Herzschrittmachers mit Hilfe wenigstens einer Sonde (58), wobei die Elektrode einen Körper (16), eine in den Körper geschraubte mechanische Schraube (20), eine mit der mechanischen Schraube fest verbundene biologische Schraube (18), die eine erste Position im Körper und eine zweite vorstehende Position einnehmen kann, und eine ummantelte Verbindungsfeder (12), die an der Elektrode befestigt ist und an ihrem freien Ende einen Verbinder (26) aufweist, umfaßt, **dadurch gekennzeichnet, daß** sie einen ergreifbaren Körper (28), der mit Kopplungsmitteln (34) versehen ist, um den Verbinder (26) der ummantelten Feder aufzunehmen und zu verriegeln, Mittel (42), die die Kopplungsmittel in bezug auf den ergreifbaren Körper (28) drehen, sowie Aufnahmemittel (56), die die Einführung der in der ummantelten Feder verwendeten Sonde (58) ermöglichen und die Sonde (58) in bezug auf den ergreifbaren Körper (28) translatorisch und rotatorisch verriegeln, um eine vorübergehende translatorische und rotatorische Verriegelung der Sonde (58) zu ermöglichen, umfaßt.

2. Vorrichtung zum Handhaben und Anordnen einer Elektrode nach Anspruch 1, **dadurch gekennzeichnet, daß** die Kopplungsmittel eine Kopplungsmuffe (34) umfassen, die an einer Hohlwelle (30) innerhalb des ergreifbaren Körpers und koaxial zu ihm befestigt ist, wobei diese Hohlwelle mit einem Bedienungsrädchen (42) verbunden ist, das von außerhalb des ergreifbaren Körpers zugänglich ist.

3. Vorrichtung zum Handhaben und Anordnen einer Elektrode nach Anspruch 2, **dadurch gekennzeichnet, daß** sie elastische Rückstellmittel (50) umfaßt, um die Hohlwelle (30) von hinten nach vorne zu ziehen, um die Kopplungsmuffe (34) gegen den ergreifbaren Körper (28) zu drücken, indem auf diese Muffe eine rotatorische Bremsung ausgeübt wird.

4. Vorrichtung zum Handhaben und Anordnen einer Elektrode nach Anspruch 3, **dadurch gekennzeichnet, daß** die elastischen Rückstellmittel (50) koaxial zu der Hohlwelle (30) angebracht sind und sich mit einer Seite auf dem Bedienungsrädchen (42) und mit der anderen auf einem Ring (52) abstützen, dessen Kontaktoberfläche mit dem Innenraum des ergreifbaren Körpers (28) verkleinert ist.

5. Vorrichtung zum Handhaben und Anordnen einer Elektrode nach Anspruch 2, **dadurch gekennzeichnet, daß** das Bedienungsrädchen (142) zusammen mit der Kopplungsmuffe (134) hergestellt ist.

6. Vorrichtung zum Handhaben und Anordnen einer Elektrode nach einem der Ansprüche 1 bis 5, **dadurch gekennzeichnet, daß** sie Mittel (36) für die Unverwechselbarkeit der Drehrichtung und der Zählrichtung umfaßt.

7. Vorrichtung zum Handhaben und Anordnen einer Elektrode nach Anspruch 6, wenn abhängig von den Ansprüchen 2 bis 5, **dadurch gekennzeichnet, daß** die Unverwechselbarkeitsmittel einen in den Innenraum des ergreifbaren Körpers (28) vorstehenden und zusammen mit diesem Körper hergestellten Vorsprung (44) sowie ein elastisches Plättchen (46), das mit dem Bedienungsrädchen (42) fest verbunden und dazu vorgesehen ist, an dem Vorsprung einzurasten, umfassen.

8. Vorrichtung zum Handhaben und Anordnen einer Elektrode nach einem der Ansprüche 2 bis 7, **dadurch gekennzeichnet, daß** die Aufnahmemittel ein U, dessen Schenkel (56) einen Griffknopf (60) einer Sonde (58) aufnehmen, sowie einen Trichter (54), der mit der Hohlwelle fest verbunden und zwischen den Schenkeln angeordnet ist, um die Einführung des Drahts (62) diese Sonde zu erleichtern, umfassen.

9. Vorrichtung zum Handhaben und Anordnen einer Elektrode nach einem der vorhergehenden Ansprüche, **dadurch gekennzeichnet, daß** die Kopplungsmittel eine Metallschraube (166) umfassen, die die Bestimmung der Ansprechschwelle der Sonde ermöglicht.

## Claims

1. Device for handling and positioning a pacemaker electrode by means of at least one stylet (58), said electrode comprising a body (16), a mechanical screw (20) screwing into said body, a biological screw (18) fixed to the mechanical screw and able to adopt a first position inside the body and a second projecting position, and a sheathed connecting spring (12) fixed to the electrode and having a connector (26) at its free end, **characterised in that** it comprises a grippable body (28) equipped with coupling means (34) for receiving and locking the connector (26) on the sheathed spring, means (42) for turning said coupling means with respect to said grippable body (28), and reception means (56) for allowing the insertion of the stylet (58) used in the sheathed spring and for translationally and rotationally locking said stylet (58) with respect to said grippable body (28) so as to allow temporary translational and rotational locking of said stylet with respect to said spring.

2. Electrode handling and positioning device according to Claim 1, **characterised in that** the coupling means comprise a coupling sleeve (34) fixed to a hollow spindle (30), internal and coaxial to the grippable body, this hollow spindle being fixed to a manoeuvring roller (42) accessible from the outside of the grippable body.

3. Electrode handling and positioning device according to Claim 2, **characterised in that** it comprises elastic return means (50) designed to pull the hollow spindle (30) from downstream to upstream so as to press the coupling sleeve (34) against the grippable body (28) by exerting rotational braking on this sleeve.

4. Electrode handling and positioning device according to Claim 3, **characterised in that** the elastic return means (50) are mounted coaxially with the hollow spindle (30) and bear on one side on the manoeuvring roller (42) and on the other on a ring (52) whose surface affording contact with the inside of the grippable body (28) is reduced.

5. Electrode handling and positioning device according to Claim 2, **characterised in that** the manoeuvring roller (142) is manufactured with the coupling sleeve (134).

6. Electrode handling and positioning device according to any one of Claims 1 to 5, **characterised in that** it comprises means (36) of foolproof determination of the direction of rotation and counting.

7. Electrode handling and positioning device according to Claim 6 when dependent on Claims 2 to 5, **characterised in that** the foolproof determination means comprise a lug (44) projecting inside the grippable body (28) and manufactured with this body, and an elastic blade (46) fixed to the manoeuvring roller (42) designed to snap onto said lug.

8. Electrode handling and positioning device according to any one of Claims 2 to 7, **characterised in that** the reception means comprise a U whose legs (56) receive a gripping knob (60) for a stylet (58), and a funnel (54), fixed to the hollow spindle, disposed between the legs, in order to facilitate the introduction of the wire (62) of this same stylet.

9. Electrode handling and positioning device according to any one of the preceding claims, **characterised in that** the coupling means comprise a metallic screw (166) so as to enable the response threshold of the sensor to be determined.
